# EUROPEAN PATENT SPECIFICATION

(11) **EP 2 282 990 B1**
(45) Date of publication and mention of the grant of the patent: **12.10.2011**
(21) Application number: 09753880.5
(22) Date of filing: 26.05.2009
(51) Int. Cl.: C07C 241/02, C07C 243/40

(54) **NEW PROCESS FOR THE PREPARATION OF 3-(2,2,2- TRIMETHYLHYDRAZINIUM) PROPIONATE DIHYDRATE**
NEUES VERFAHREN ZUR HERSTELLUNG VON 3-(2,2,2-TRIMETHYLHYDRAZINIUM) PROPIONAT-DIHYDRAT
NOUVEAU PROCEDE POUR LA PREPARATION DE PROPIONATE DE 3-(2,2,2- TRIMETHYLHYDRAZINIUM) DIHYDRATE

(30) Priority: 26.05.2008 EP 08156903; 18.09.2008 EP 08164564
(43) Date of publication of application: 16.02.2011
(73) Proprietor: Grindeks, a joint stock company, 1057 Riga (LV)
(72) Inventor: ZICANE, Daina, 1048 Riga (LV); TURKS, Maris, 1048 Riga (LV)
(86) International application number: PCT/EP2009/056379
(87) International publication number: WO 2009/144225

(56) References cited:
- WO-A1-80/01068
- WO-A1-2005/012233
- WO-A1-2008/028514

## Description

### Technical Field

The present invention relates to an improved process for preparation of 3-(2,2,2-trimethylhydrazinium)propionate dihydrate (international non-proprietary name-"Meldonium").

### Background Art

A number of processes for the preparation of 3-(2,2,2-trimethylhydrazinium)propionate dihydrate is known.

The first process for preparation 3-(2,2,2-trimethylhydrazinium)propionate dihydrate is disclosed in WO 80/01068 A (INST ORGANICHESKOGO SINTEZA) 1980.05.29.. The process starts with methyl 3-(2,2-dimethylhydrazino)propionate is treated with a methyl halide or dimethylsulphate to give the appropriate trimethylhydrazinium salt, which is transferred to 3-(2,2,2-trimethylhydrazinium)propionate by Amberlite IRA-400 (OH form). After crystallization from ethanol the inner salt is obtained as a dihydrate.

This process disclosed in above patent can be considered as a common process for 3-(2,2,2-trimethylhydrazinium)propionate dihydrate preparation. The method has many disadvantages: strongly basic ion exchangers are unstable and undergo decomposition and oxidation during processing; they withstand only a limited number of regeneration cycles; large quantities or solvents, acids and bases as well as deionised water are needed to regenerate the resins; low ion exchange capacity and therefore high production costs of 3-(2,2,2-trimethylhydrazinium)propionate dihydrate by this process are typical. This process is not convenient for large scale production of 3-(2,2,2-trimethylhydrazinium)propionate dihydrate.

A standard method of alkaline hydrolysis of carbonic acid ester in case of an 3-(2,2,2-trimethylhydrazinium)propionate salt could not be successfully realised because of the problems of separation of 3-(2,2,2-trimethylhydrazinium)propionate dihydrate and the resulting inorganic salts. It is known that 3-(2,2,2-trimethylhydrazinium)propionate forms various double salts, some of them as well is disclosed in SU 978808 (INST ORGANICHESKOGO SINTEZA) 07.12.1982.

The above mentioned technical problem was tried to solve in WO 2008/028514 A (SILVA JORGE) 2008.03.13. , which disclosed a method for producing 3-(2,2,2-trimethylhydrazinium)propionate dihydrate by hydrolyzed under acidic conditions with catalysis by HCl, sulphuric acid, phosphoric acid etc., esters of 3-(2,2,2-trimethylhydrazinium)propionate halide or methyl sulphate followed by neutralisation by an appropriate inorganic base (for example-sodium, potassium, calcium or magnesium hydroxide or another appropriate base, for example sodium, potassium, lithium or caesium carbonate or bicarbonate etc.) and the double salts thus obtained can be separated by the invented process using saturation the solution with carbon dioxide or sulphur dioxide.

Nevertheless method disclosed in WO 2008/028514 A (SILVA JORGE) 2008.03.13. allow to avoid use of electrodialysis in preparation 3-(2,2,2-trimethylhydrazinium)propionate dihydrate, but at the same time double salts are formed and then have to follow process step for separation of double salts, in this case it is saturation the solution with carbon dioxide or sulphur dioxide.

### Disclosure of Invention

The above objective is achieved according to present invention by hydrolysis of methyl-3-(2,2,2-trimethylhydrazinium)propionate methylsulphate to yield crude 3-(2,2,2-trimethylhydrazinium)propionate dihydrate; crystalization of crude 3-(2,2,2-trimethylhydrazinium)propionate dihydrate with mixture of ethanol/water solution or isopropanol/water.

### Advantageous Effects of the Invention

The following Reaction Scheme 1 outlines the present method of preparation 3-(2,2,2-trimethylhydrazinium)propionate dihydrate. The method includes the following advantages:
The main effort of present invention disclosed in when methyl-3-(2,2,2-trimethylhydrazinium)propionate methylsulphate was hydrolyzed with calcium hydroxide in ethanolic mixture, by obtaining calcium sulphate dihydrate residue, which is poorly soluble in water/ethanol and fall quantitative into the residue.

Thereby not formed complexes or double salts with methyl-3-(2,2,2-trimethylhydrazinium)propionate, what is the main problem by using correspond halides of compound methyl-3-(2,2,2-trimethylhydrazinium)propionate. Residue of calcium sulphate dihydrate is easily remove by filtration and filtrate contains crude product of 3-(2,2,2-trimethylhydrazinium)propionate dihydrate. No further electrodialysis is necessary.

Calcium hydroxide as such for preparation 3-(2,2,2-trimethylhydrazinium)propionate dihydrate is mentioned in WO 2008/028514 A (SILVA JORGE) 2008.03.13, but it is just mentioned in description and in the claims, and expected result is what forms double salts, which thereafter will be can be separated by using saturation the solution with carbon dioxide or sulphur dioxide, person skilled in the art can't expect what no double salt is formed and no further saturation the solution with carbon dioxide or sulphur dioxide is not necessary.

This process is amenable to large scale production which does not require specialized equipment.

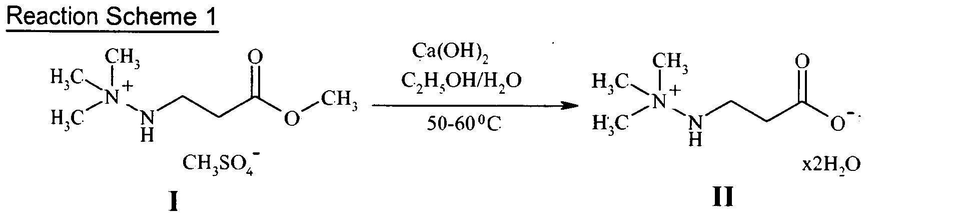

Thus, the subject matter of the present invention is a process for preparing a compound of formula II:

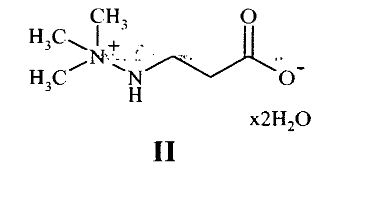

The process comprising the following:
hydrolysis compound of formula I 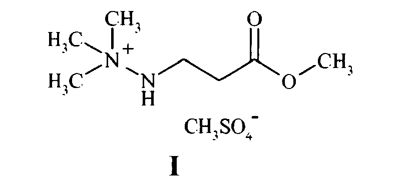 with calcium hydroxide to form crude 3-(2,2,2-trimethylhydrazinium)propionate dihydrate of formula II; and 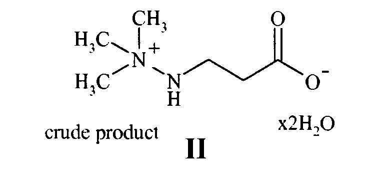 crystallization crude product from water/ethanol solution or water/isopropanol for obtaining the desired compound 3-(2,2,2-trimethylhydrazinium)propionate dihydrate.

### Best Mode for Carrying Out the Invention

The present invention will be described in more detail by referring to the following examples.

### Example

The crude emulsive methyl-3-(2,2,2-trimethylhydrazinium) propionate methylsulphate was dissolved in distilled water (500 mL) and ethanol (200mL, 96%). Alkaline agent was added to stirred reaction mixture.

Great list of alkaline agents were used, almost all of them formed double salts, surprisingly just only calcium hydroxide didn't form any double salt.

| Alkaline agent | Result |
|---|---|
| Sodium oxide | Form a double salt |
| Potassium oxide | Form a double salt |
| Lithium oxide | Form a double salt |
| Caesium oxide | Form a double salt |
| Calcium oxide | Form a double salt |
| Magnesium oxide | Form a double salt |
| Sodium hydroxidee | Form a double salt |
| Potassium hydroxide | Form a double salt |
| Lithium hydroxide | Form a double salt |
| Caesium hydroxide | Form a double salt |
| Calcium hydroxide | No double salt was formed |
| Magnesium hydroxide | Form a double salt |
| Sodium carbonate | Form a double salt |
| Potassium carbonate | Form a double salt |
| Lithium carbonate | Form a double salt |
| Caesium carbonate | Form a double salt |
| Calcium carbonate | Form a double salt |
| Magnesium carbonate | Form a double salt |
| Sodium bicarbonate | Form a double salt |
| Potassium bicarbonate | Form a double salt |
| Lithium bicarbonate | Form a double salt |
| Caesium bicarbonate | Form a double salt |
| Calcium bicarbonate | Form a double salt |
| Magnesium bicarbonate | Form a double salt |

### Example 1

### Preparation of crude 3-(2,2,2-trimethylhydrazinium)propionate dihydrate

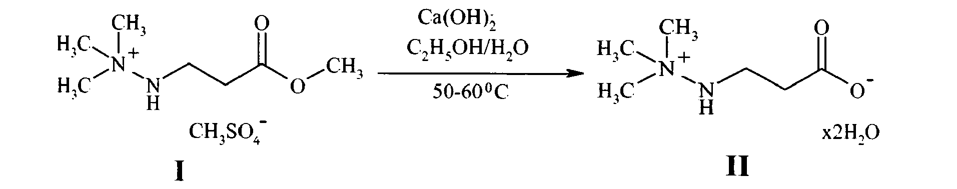

The crude emulsive methyl-3-(2,2,2-trimethylhydrazinium) propionate methylsulphate was dissolved in distilled water (500 mL) and ethanol (200 ml, 96%). Calcium hydroxide (54.2 g, 0.73 mol) was added to stirred reaction mixture. The reaction mixture was stirred and heated to 50-60°C for 2 hours. The reaction process was controlled by TLC.

Thereafter the reaction mixture was filtered to remove calcium sulphate dihydrate. The calcium sulphate dihydrate cake on the filter was washed with ethanol (100 mL).

The filtrate of reaction mixture was concentrated in vacuo, then isopropanol (50 mL), tert-butylmethylether (20 mL) and pure 3-(2,2,2-trimethylhydrazinium)propionate dihydrate as crystallization germ (0.5 g) were added to reaction mixture. The obtained crystallization mixture was stirred at - 15-(-5) °C. The suspension was filtered; the crude 3-(2,2,2-trimethylhydrazinium)propionate dihydrate was washed with tert-butylmethylether (150 mL) on the filter.

### Example 2

### Preparation of 3-(2,2,2-trimethylhydrazinium)propionate dihydrate

The crude 3-(2,2,2-trimethylhydrazinium)propionate dihydrate (162 g) was added to stirred in mixture of ethanol (550 mL)/water(20 mL) or in mixture of isopropanol/water. The reaction mixture was heated to 70°C for 20 minutes till all crude product was dissolved, at which point it was filtered.

The filtrate was cooled to -10°C for 2 hours. The precipitate was separated by filtration. The yield 3-(2,2,2-trimethylhydrazinium)propionate dihydrate was 99.0g (90%) of white crystalline powder.

## Claims

1. A process for preparing 3-(2,2,2-trimethylhydrazinium)propionate dihydrate of formula II 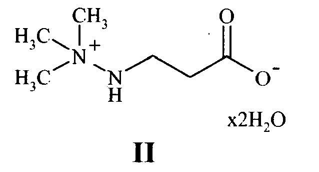 comprising hydrolysis the compound of formula I 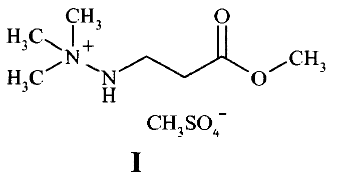 carried out with calcium hydroxide;

## Patentansprüche

1. Verfahren zur Herstellung von 3-(2,2,2-trimethylhydrazinium)propionatdihydrat gemäß Formel II 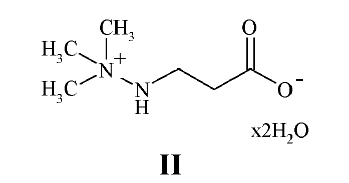 umfassend die Hydrolyse von Verbindung gemäß Formel I 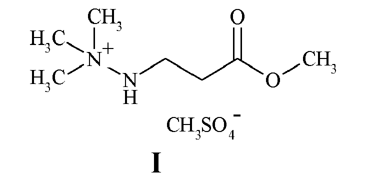 mit Calciumhydroxid;

## Revendications

1. Processurs pour préparer 3-(2,2,2-triméthylhydrazine) propionate dihydrate de la formule II 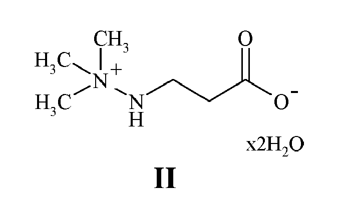 contenant l'hydrolyse du composé de la formule I 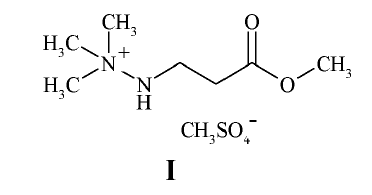 effectuée avec hydroxyde de calcium
